# EUROPEAN PATENT APPLICATION

(11) **EP 1 867 341 A1**
(43) Date of publication of application: **19.12.2007**
(21) Application number: 06730206.7
(22) Date of filing: 28.03.2006
(51) Int. Cl.: A61K 45/00, A61P 1/16, A61P 43/00, G01N 33/15, G01N 33/50, G01N 33/566

(54) **THERAPEUTIC AGENT FOR NON-ALCOHOLIC FATTY LIVER DISEASE, AND SCREENING METHOD FOR DRUG CANDIDATE COMPOUND FOR TREATMENT OR PREVENTION OF NON-ALCOHOLIC FATTY LIVER DISEASE**

(30) Priority: 29.03.2005 JP 2005095064; 04.10.2005 JP 2005291536
(71) Applicant: BANYU PHARMACEUTICAL CO., LTD., Tokyo 102-8667 (JP)
(72) Inventor: GOMORI, Akira, Tsukuba-shi, Ibaraki 300-2611 (JP); ISHIHARA, Akane, Tsukuba-shi, Ibaraki 300-2611 (JP); IWAASA, Hisashi, Tsukuba-shi, Ibaraki 300-2611 (JP); SUZUKI, Jun, Tsukuba-shi, Ibaraki 300-2611 (JP); ITO, Makoto, Tsukuba-shi, Ibaraki 300-2611 (JP); KANATANI, Akio, Tsukuba-shi, Ibaraki 300-2611 (JP)
(74) Representative: Hussain, Deeba
(86) International application number: PCT/JP2006/306258
(87) International publication number: WO 2006/104136

(57) **Abstract**

A therapeutic agent for a non-alcoholic fatty liver disease comprising a melanin-concentrating hormone receptor antagonist as an active ingredient, which is developed based on a novel mechanism of action that a melanin-concentrating hormone receptor is involved in non-alcoholic fatty liver diseases. A method for screening a drug candidate compound for the treatment or prevention of a non-alcoholic fatty liver disease by utilizing the mechanism.

## Description

### TECHNICAL FIELD

The present invention relates to a therapeutic agent for a non-alcoholic fatty liver disease. The invention also relates to a method for screening a drug candidate compound for the treatment or prevention of a non-alcoholic fatty liver disease.

### BACKGROUND ART

Fatty liver diseases are roughly divided into two groups: one is attributable to alcohol intake, and the other is not attributable thereto. The latter is called a non-alcoholic fatty liver disease (NAFLD) including, for example, non-alcoholic fatty liver and non-alcoholic steatohepatitis (NASH).

It has been reported that PPARα agonist (Non-patent document 1) or metformin (Non-patent document 2) is effective in NAFLD, however, both have side effects which cannot be ignored.

On the other hand, a melanin-concentrating hormone (MCH) is considered to be a factor which induces eating behavior (an appetite-stimulating hormone) (for example, see Non-patent documents 3 to 5), however, there has been no knowledge of the relationship between fatty liver or hepatitis and MCH.

Non-patent document 1: Basaranoglu M. et al., J. Hepatology, vol. 31, pp. 384 (1999)
Non-patent document 2: Marchesini G. et al., Lancet, vol. 358, pp. 893 (2001)
Non-patent document 3: Masako Shimada "The Role of Melanin-Concentrating Hormone (MCH) in Obesity", Saishin Igaku, vol. 56, pp. 121-127 (2001)
Non-patent document 4: Chambers J. et al., Nature, vol. 400, pp. 261 (1999)
Non-patent document 5: Saito Y. et al., Nature, vol. 400, pp. 265 (1999)

### DISCLOSURE OF THE INVENTION

### PROBLEMS THAT THE INVENTION IS TO SOLVE

As described above, there has not yet been a drug which is sufficient to be used as a therapeutic agent for NAFLD. If a therapeutic agent for NAFLD based on a novel mechanism of action can be developed, it will be possible to expand the choice of treatment of NAFLD. Thus, an object of the invention is to provide a therapeutic agent for NAFLD based on a novel mechanism of action. Further, another object of the invention is to provide a method for screening a drug candidate compound for the treatment or prevention of NAFLD based on a novel mechanism of action.

### MEANS FOR SOLVING THE PROBLEMS

The present inventors found that a melanin-concentrating hormone receptor antagonist has actions of suppressing fatty liver and suppressing inflammation, and thus developed a therapeutic agent for NAFLD based on a novel mechanism of action.

That is, the invention provides the following therapeutic agent for NAFLD.
(1) A therapeutic agent for a non-alcoholic fatty liver disease comprising a melanin-concentrating hormone receptor antagonist as an active ingredient.
(2) The therapeutic agent according to (1), wherein the non-alcoholic fatty liver disease is non-alcoholic fatty liver.
(3) The therapeutic agent according to (1), wherein the non-alcoholic fatty liver disease is non-alcoholic steatohepatitis.
(4) The therapeutic agent according to any of (1) to (3), wherein the melanin-concentrating hormone receptor antagonist is a melanin-concentrating hormone receptor 1 antagonist.

Further, the invention provides the following method for screening a drug candidate compound for the treatment or prevention of NAFLD.
(5) A method for screening a drug candidate compound for the treatment or prevention of a non-alcoholic fatty liver disease comprising the steps of:
   (a) bringing a test compound into contact with a melanin-concentrating hormone receptor;
   (b) detecting binding of the test compound to the melanin-concentrating hormone receptor; and
   (c) selecting the test compound binding to the melanin-concentrating hormone receptor.
(6) A method for screening a drug candidate compound for the treatment or prevention of a non-alcoholic fatty liver disease comprising the steps of:
   (a) bringing a test compound into contact with a cell which expresses a melanin-concentrating hormone receptor;
   (b) measuring the expression level of the melanin-concentrating hormone receptor; and
   (c) selecting the test compound which decreases the expression level of the melanin-concentrating hormone receptor in comparison with the case where the test compound is not brought into contact.
(7) A method for screening a drug candidate compound for the treatment or prevention of a non-alcoholic fatty liver disease comprising the steps of:
   (a) providing a cell or a cell extract having a DNA in which a reporter gene is functionally linked downstream of a promoter region of a DNA encoding a melanin-concentrating hormone receptor;
   (b) bringing a test compound into contact with the cell or cell extract;
   (c) measuring the expression level of the reporter gene in the cell or cell extract; and
   (d) selecting the test compound which decreases the expression level of the reporter gene in comparison with the case where the test compound is not brought into contact.
(8) A method for screening a drug candidate compound for the treatment or prevention of a non-alcoholic fatty liver disease comprising the steps of:
   (a) bringing a test compound into contact with a cell which expresses a melanin-concentrating hormone receptor on a cell surface in the presence of a ligand for the melanin-concentrating hormone receptor;
   (b) measuring the activity of the melanin-concentrating hormone receptor in the cell; and
   (c) selecting the test compound which decreases the activity of the melanin-concentrating hormone receptor in comparison with the case where the test compound is not brought into contact.
(9) The therapeutic agent according to any of (5) to (8), wherein the non-alcoholic fatty liver disease is non-alcoholic fatty liver.
(10) The therapeutic agent according to any of (5) to (8), wherein the non-alcoholic fatty liver disease is non-alcoholic steatohepatitis.
(11) The screening method according to any of (5) to (10), wherein the melanin-concentrating hormone receptor is melanin-concentrating hormone receptor 1.

### ADVANTAGE OF THE INVENTION

The invention provides a therapeutic agent for NAFLD based on a novel mechanism of action, and can expand the choice of treatment of NAFLD.

### BRIEF DESCRIPTION OF THE DRAWINGS

[Fig. 1] Fig. 1 is a drawing showing an effect of Compound A on DIO mice. Fig. 1(a) shows the liver weight, and Fig. 1(b) shows the plasma ALT.
[Fig. 2] Fig. 2 is a drawing showing an effect of Compound A (plasma AST level) on NASH mice induced by MCD diet.
[Fig. 3] Fig. 3 is a drawing showing an effect of Compound A (hepatic triglyceride level) on NASH mice induced by MCD diet.
[Fig. 4] Fig. 4 is a drawing showing typical images of liver pathology of NASH mice induced by MCD diet. Fig. 4(a) shows a vehicle administration group, and Fig. 4(b) shows a Compound A administration group.
[Fig. 5] Fig. 5 is a drawing showing an effect of Compound A (plasma ALT level) on NASH mice induced by MCD diet.
[Fig. 6] Fig. 6 is a drawing showing an effect of Compound A on NASH mice induced by MCD diet. Fig. 6(a) shows the TBARS level, and Fig. 6(b) shows the expression level of Cyp4A14.
[Fig. 7] Fig. 7 is a drawing showing an effect of Compound A on NASH mice induced by MCD diet. Fig. 7(a) shows the expression level of TNFα, and Fig. 7(b) shows the expression level of IL-1β.
[Fig. 8] Fig. 8 is a drawing showing an effect of Compound A on NASH mice induced by HFD.
Fig. 8(a) shows the hepatic triglyceride level, Fig. 8(b) shows the plasma ALT level, and Fig. 8(c) shows the plasma AST level.
[Fig. 9] Fig. 9 is a drawing showing typical images of liver pathology of NASH mice induced by HFD. Fig. 9(a) shows a vehicle administration group, and Fig. 9(b) shows a Compound A administration group.

### BEST MODE FOR CARRYING OUT THE INVENTION

The therapeutic agent for NAFLD of the invention is characterized by containing a melanin-concentrating hormone (MCH) receptor antagonist as an active ingredient. NAFLD includes non-alcoholic fatty liver, non-alcoholic steatohepatitis and the like.

The MCH receptor antagonist which is an active ingredient of the therapeutic agent may be any as long as it inhibits the activity of the MCH receptor, and a lot of antagonists have already been known. Specific examples thereof include SNAP-7941, T-226296 and the like. These antagonists can be produced based on a known method.

It is known that there exist melanin-concentrating hormone receptor 1 (MCH1R) and melanin-concentrating hormone receptor 2 (MCH2R) in the MCH receptors. An MCH1R antagonist is excellent in its actions of suppressing fatty liver and suppressing inflammation, therefore, the MCH receptor antagonist which is an active ingredient of the invention is preferably an MCH1R antagonist. Examples of the MCH1R antagonist include SNAP-7941, T-226296 and the like.

The therapeutic agent for NAFLD of the invention can be formulated into various preparations by adding a pharmaceutically acceptable additive to the MCH receptor antagonist according to its dosage form. As the additive, any of various additives which are conventionally used in the field of pharmaceuticals can be used, and examples thereof include gelatin, lactose, sucrose, titanium oxide, starch, crystalline cellulose, hydroxypropylmethyl cellulose, carboxymethyl cellulose, corn starch, microcrystalline wax, white soft paraffin, magnesium aluminometasilicate, anhydrous calcium phosphate, citric acid, trisodium citrate, hydroxypropyl cellulose, sorbitol, sorbitan fatty acid esters, polysorbate, sucrose fatty acid esters, polyoxyethylene, hydrogenated castor oil, polyvinyl pyrrolidone, magnesium stearate, light anhydrous silicic acid, talc, vegetable oils, benzyl alcohol, gum arabic, propylene glycol, polyalkylene glycol, cyclodextrin and hydroxypropyl cyclodextrin and the like.

Examples of the dosage form to be formulated as a mixture with any of these additives include solid preparations such as a tablet, a capsule, a granule, a powder and a suppository, liquid preparations such as a syrup, an elixir and an injection, and the like. These preparations can be prepared in accordance with a conventional method in the field of pharmaceuticals. In this connection, in the case of the liquid preparation, it may be in a form which is dissolved or suspended in water or other suitable solvent before use. Also, particularly in the case of an injection, it may be dissolved or suspended in a physiological saline solution or a glucose solution according to need or further mixed with a buffer or a preservative.

In the case where the therapeutic agent for NAFLD of the invention is used in, for example, a clinical field, its dose and administration frequency vary depending on the patient's sex, age, body weight, the severity of symptoms, and the type and range of the intended therapeutic effect and the like. However, in general, in the case of oral administration, it is administered to an adult in an amount of from 0.01 to 100 mg/kg per day, preferably from 0.03 to 1 mg/kg per day in terms of the MCH receptor antagonist by dividing the daily dose into 1 to several times. In the case of parenteral administration, it is administered in an amount of from 0.001 to 10 mg/kg per day, preferably from 0.001 to 0.1 mg/kg per day, more preferably from 0.01 to 0.1 mg/kg per day in terms of the MCH receptor antagonist by dividing the daily dose into 1 to several times. General physicians, veterinarians or clinicians can easily determine the effective dose of the drug which is required to prevent, suppress or stop the progress of pathology and provide a treatment.

The therapeutic agent for NAFLD of the invention can contain the MCH receptor antagonist in an amount of from 1.0 to 100% by weight, preferably from 1.0 to 60% by weight of the total preparation. These pharmaceutical preparations may also contain any other therapeutically effective compounds.

Subsequently, the screening method of the invention will be described. In a first embodiment of the screening method of the invention, first, a test compound is brought into contact with a melanin-concentrating hormone receptor.

The nucleotide sequence of cDNA of human-derived melanin-concentrating hormone receptor 1 is represented by SEQ ID NO: 1, and the amino acid sequence of the protein encoded by the cDNA is represented by SEQ ID NO: 2. The nucleotide sequence of cDNA of mouse-derived melanin-concentrating hormone receptor 1 is represented by SEQ ID NO: 3, and the amino acid sequence of the protein encoded by the cDNA is represented by SEQ ID NO: 4.

Further, in the melanin-concentrating hormone receptor to be used in the screening method of the invention, proteins functionally equivalent to the melanin-concentrating hormone receptor are included. Examples of such a protein include mutants, alleles, variants, and homologs of melanin-concentrating hormone receptor, partial peptides of melanin-concentrating hormone receptor, fusion proteins with other proteins and the like, however, it is not limited to these. Further, in place of the melanin-concentrating hormone receptor to be used in the screening method of the invention, a cell or a tissue expressing the melanin-concentrating hormone receptor can also be used. Examples of such a tissue include animal tissues (for example, brain, fat, and liver), and examples of such a cell include cells derived from the animal tissues. Animal species from which animal tissues or cells are isolated is not particularly limited, and examples thereof include humans, monkeys, dogs, rabbits, rats, mice, and ferrets.

In the invention, as the mutant of melanin-concentrating hormone receptor, a protein which is a naturally occurring protein composed of an amino acid sequence in which one or more amino acids are substituted, deleted, inserted and/or added in the amino acid sequence represented by SEQ ID NO: 2 or 4, and is functionally equivalent to the protein composed of the amino acid sequence represented by SEQ ID NO: 2 or 4 can be exemplified. Further, a protein which is encoded by a naturally occurring DNA hybridized to the DNA composed of the nucleotide sequence represented by SEQ ID NO: 1 or 3 under stringent conditions and is functionally equivalent to the protein composed of the amino acid sequence represented by SEQ ID NO: 2 or 4 can also be exemplified as the mutant of melanin-concentrating hormone receptor.

In the invention, the number of amino acids to be mutated is not particularly limited, however, it is considered to be generally 30 amino acids or less, preferably 15 amino acids or less, more preferably 5 amino acids or less (for example, 3 amino acids or less). With regard to the amino acid residue to be mutated, it is desirable that the amino acid is mutated to another amino acid in which the property of the amino acid side chain is conserved. For example, with regard to the property of the amino acid side chain, hydrophobic amino acids (A, I, L, M, F, P, W, Y, V), hydrophilic amino acids (R, D, N, C, E, Q, G, H, K, S, T), amino acids having an aliphatic side chain (G, A, V, L, I, P), amino acids having a hydroxyl group-containing side chain (S, T, Y), amino acids having a sulfur atom-containing side chain (C, M), amino acids having a carboxylic acid and amide-containing side chain (D, N, E, Q), amino acids having a base-containing side chain (R, K, H), and amino acids having an aromatic- containing side chain (H, F, Y, W) can be exemplified (the parenthetic letters indicate the one-letter codes of amino acids). It has been already known that a polypeptide having an amino acid sequence modified by deletion, addition, and/or substitution with another amino acid of one or more amino acid residues in a given amino acid sequence retains the biological activity of the original polypeptide.

The "functionally equivalent" in the invention refers to that a subject protein has a biological function or a biochemical function equivalent to that of melanin-concentrating hormone receptor. In the invention, as the biological function or biochemical function of melanin-concentrating hormone receptor, binding to a melanin-concentrating hormone and the like can be exemplified. In the biological function, specificity of the site to be expressed, an expression level and the like are also included.

As methods well known to those skilled in the art in order to prepare a DNA encoding the "protein functionally equivalent" to the target protein, methods utilizing a hybridization technique and a polymerase chain reaction (PCR) technique can be exemplified. That is, for those skilled in the art, isolation of a DNA with a high homology to the melanin-concentrating hormone receptor by using the nucleotide sequence of melanin-concentrating hormone receptor (SEQ ID NO: 1 or 3) or a partial sequence thereof as a probe, and by using an oligonucleotide specifically hybridized to the nucleotide sequence of melanin-concentrating hormone receptor (SEQ ID NO: 1 or 3) as a primer can be conventionally carried out. The DNA encoding the protein having a function equivalent to the melanin-concentrating hormone receptor that can be isolated by way of hybridization technique and PCR technique as in the above is also included in the DNA of the invention.

In order to isolate such a DNA, the hybridization reaction is preferably carried out under stringent conditions. The stringent hybridization conditions in the invention refer to conditions of 6 M urea, 0.4% SDS, and 0.5 × SSC, or hybridization conditions as stringent as the above conditions. It can be expected that by employing more stringent conditions, for example, conditions of 6 M urea, 0.4% SDS, and 0.1 × SSC, a DNA with a higher homology is isolated. It is considered that the DNA isolated in this way has a high homology to the amino acid sequence of the target protein at the amino acid level. The high homology refers to at least 50% or more, preferably 70% or more, and more preferably 90% or more (for example, 95%, 96%, 97%, 98%, 99% or more) sequence identity in the entire amino acid sequence. The identity of an amino acid sequence or a nucleotide sequence can be determined by using the algorithm BLAST of Karlin and Altschul (Proc. Natl. Acad. Sci. USA 87: 2264-2268, 1990, Proc. Natl. Acad. Sci. USA 90: 5873, 1993). The programs called BLASTN and BLASTX based on the algorithm of BLAST have been developed (Altschul SF, et al.: J. Mol. Biol. 215: 403, 1990). In the case where a nucleotide sequence is analyzed using BLASTN, for example, the parameters are set as follows: score = 100, wordlength = 12. Further, in the case where an amino acid sequence is analyzed using BLASTX, for example, the parameters are set as follows: score = 50, wordlength = 3. In the case where BLAST and Gapped BLAST program are used, default parameters of the respective programs are used. Specific techniques of these analysis methods are known.

The biological species from which the melanin-concentrating hormone receptor to be used in the method of the invention is not particularly limited to a specific biological species, and examples thereof include humans, monkeys, mice, rats, guinea pigs, pigs, cattle, yeasts, insects and the like.

The state of melanin-concentrating hormone receptor to be used in the first embodiment is not particularly limited, and for example, it may be a purified state, a state of being expressed in a cell, a state of being expressed in a cell extract, or the like.

Purification of melanin-concentrating hormone receptor can be carried out by a known method. Further, examples of the cell expressing a melanin-concentrating hormone receptor include a cell expressing an endogenous melanin-concentrating hormone receptor and a cell expressing an exogenous melanin-concentrating hormone receptor. Examples of the cell expressing an endogenous melanin-concentrating hormone receptor include cultured cells and the like, however, it is not limited to these. The cultured cells are not particularly limited, and for example, commercially available one can be used. The biological species from which the cell expressing an endogenous melanin-concentrating hormone receptor is derived is not particularly limited, and examples thereof include humans, monkeys, mice, rats, guinea pigs, pigs, cattle, yeasts, insects and the like. Further, the cell expressing an exogenous melanin-concentrating hormone receptor can be produced by, for example, introducing a vector containing a DNA encoding a melanin-concentrating hormone receptor into a cell. The introduction of the vector into a cell can be carried out by a general method, for example, a calcium phosphate precipitation method, an electric pulse electroporation method, a lipofectamine method, a microinjection method, or the like. Further, the cell having an exogenous melanin-concentrating hormone receptor can be produced by, for example, inserting a DNA encoding a melanin-concentrating hormone receptor into a chromosome by a gene transfer method utilizing homologous recombination. The biological species from which such a cell transfected with an exogenous melanin-concentrating hormone receptor is derived is not limited to a mammal, and can be any as long as it is a biological species for which a technique of expressing an exogenous protein in a cell is established.

Further, as the cell extract in which a melanin-concentrating hormone receptor is expressed, a cell extract obtained by adding a vector containing a DNA encoding a melanin-concentrating hormone receptor to a cell extract contained in an in vitro transcription/ translation system can be exemplified. The in vitro transcription/translation system is not particularly limited, and a commercially available in vitro transcription/translation kit or the like can be used.

The "test compound" in the method of the invention is not particularly limited, and examples thereof include single compounds such as natural compounds, organic compounds, inorganic compounds, proteins and peptides, compound libraries, expression products of gene libraries, cell extracts, cell culture supernatants, fermented microorganism products, marine organism extracts, plant extracts, prokaryotic cell extracts, eukaryotic single cell extracts, animal cell extracts and the like. Such a test sample can be used by appropriately labeling if necessary. As the labeling, for example, radiolabeling, fluorescent labeling and the like can be exemplified. Further, in addition to the above test samples, a mixture obtained by mixing plural types of these test samples is also included.

Further, the "contact" in the invention is carried out according to the state of the melanin-concentrating hormone receptor. For example, when the melanin-concentrating hormone receptor is in a purified state, the contact can be carried out by adding a test sample to a purified preparation. Further, when it is in a state of being expressed in a cell, or a state of being expressed in a cell extract, the contact can be carried out by adding a test sample to a cell culture solution or an extract of the cell. In the case where the test sample is a protein, for example, the contact can also be carried out by introducing a vector containing a DNA encoding the protein into a cell expressing a melanin-concentrating hormone receptor, or adding the vector to a cell extract expressing a melanin-concentrating hormone receptor. Further, for example, the contact can also be carried out by utilizing a two-hybrid method with the use of a yeast, an animal cell or the like.

In the first embodiment, subsequently, the binding of the test compound to the melanin-concentrating hormone receptor is detected. The detection method is not particularly limited. The binding of the test compound to the melanin-concentrating hormone receptor can be detected by, for example, a label (for example, a label which can be quantitatively measured such as a radiolabel or a fluorescent label) attached to the test compound bound to the melanin-concentrating hormone receptor. Further, the detection can also be carried out by using a change in the activity of melanin-concentrating hormone receptor caused by the binding of the test compound to the melanin-concentrating hormone receptor as an index.

In this embodiment, subsequently, the test compound binding to the melanin-concentrating hormone receptor is selected. In the selected compounds, a compound which suppresses the activity of the melanin-concentrating hormone receptor or a compound which decreases the expression of the melanin-concentrating hormone receptor is included.

In a second embodiment of the screening method of the invention, first, a test compound is brought into contact with a cell expressing a melanin-concentrating hormone receptor.

In the second embodiment, subsequently, the expression level of the melanin-concentrating hormone receptor is measured. The measurement of the expression level of the melanin-concentrating hormone receptor can be carried out by a method known to those skilled in the art. For example, mRNA of melanin-concentrating hormone receptor gene is extracted according to a standard method, and the transcription level of the gene can be measured by a Northern hybridization method or an RT-PCR method with the use of this mRNA as a template. Further, by using a DNA array technique, the expression level of the gene can also be measured.

Further, the measurement of the gene at a translation level can also be carried out by recovering a fraction containing the melanin-concentrating hormone receptor encoded by the melanin-concentrating hormone receptor gene according to a standard method, and detecting the expression of the melanin-concentrating hormone receptor by electrophoresis such as SDS-PAGE. Further, the measurement of the gene at a translation level can also be carried out by performing a Western blotting method with the use of an antibody against the melanin-concentrating hormone receptor, and detecting the expression of the melanin-concentrating hormone receptor.

The antibody to be used in the detection of the melanin-concentrating hormone receptor is not particularly limited as long as it is an antibody which can be detected, however, for example, both of a monoclonal antibody and a polyclonal antibody can be used. The antibody can be prepared by a method known to those skilled in the art. In the case of the polyclonal antibody, it can be obtained, for example, as follows. A small animal such as a rabbit is immunized with a melanin-concentrating hormone receptor or a recombinant protein or a partial peptide thereof, which has been expressed in a microorganism such as E. coli as a fusion protein with GST, and the serum is obtained. Then, the obtained serum is purified by, for example, ammonium sulfate precipitation, a protein A or protein G column, DEAE ion exchange chromatography, an affinity column coupled with the melanin-concentrating hormone receptor or a synthetic peptide or the like, whereby the polyclonal antibody is prepared. In addition, in the case of the monoclonal antibody, for example, a small animal such as a mouse is immunized with a melanin-concentrating hormone receptor or a partial peptide thereof, the spleen is removed from the mouse. Then, the spleen is homogenized and cells are separated. The separated cells and the mouse myeloma cells are fused using a reagent such as polyethylene glycol, and from the thus obtained fusion cells (hybridomas), a clone which produces an antibody capable of binding to the melanin-concentrating hormone receptor is selected. Subsequently, the obtained hybridoma is transplanted into the abdominal cavity of a mouse, the ascitic fluid is collected from the mouse, and the obtained monoclonal antibody is purified by, for example, ammonium sulfate precipitation, a protein A or protein G column, DEAE ion exchange chromatography, an affinity column coupled with the melanin-concentrating hormone receptor or a synthetic peptide or the like, whereby the preparation thereof can be achieved.

In the second embodiment, subsequently, the test compound which decreases the expression level of the melanin-concentrating hormone receptor in comparison with the case where the test compound is not brought into contact is selected. In the selected compounds, a compound which decreases the expression of the melanin-concentrating hormone receptor is included.

In a third embodiment of the screening method of the invention, first, a cell or a cell extract having a DNA in which a reporter gene is functionally linked downstream of a promoter region of a DNA encoding a melanin-concentrating hormone receptor is provided.

In the third embodiment, the "functionally linked" refers to that a reporter gene is linked to a promoter region of melanin-concentrating hormone receptor gene such that the expression of the reporter gene is induced by the linking of a transcription factor to the promoter region of melanin-concentrating hormone receptor gene. Accordingly, the case, in which even if the reporter gene is linked to other gene and a fusion protein with other gene product is formed, the expression of the fusion protein is induced by the linking of a transcription factor to the promoter region of melanin-concentrating hormone receptor gene, is included in the meaning of the "functionally linked".

The reporter gene is not particularly limited as long as the expression thereof can be detected, and examples thereof include a CAT gene, a lacZ gene, a luciferase gene, a β-glucuronidase gene (GUS), a GFP gene and the like, which are conventionally used by those skilled in the art. Further, in the reporter gene, a DNA encoding a melanin- concentrating hormone receptor protein is also included.

The cell or cell extract having a DNA in which a reporter gene is functionally linked downstream of a promoter region of a DNA encoding a melanin-concentrating hormone receptor can be prepared by the method described in the first embodiment.

In the third embodiment, subsequently, a test sample is brought into contact with the cell or cell extract. Then, the expression level of the reporter gene in the cell or cell extract is measured.

The expression level of the reporter gene can be measured by a method known to those skilled in the art according to the type of the reporter gene to be used. For example, in the case where the reporter gene is a CAT gene, the expression level of the reporter gene can be measured by detecting the acetylation of chloramphenicol caused by the gene product. In the case where the reporter gene is a lacZ gene, by detecting the coloring of a pigment compound caused by the catalytic action of the gene expression product, in the case where the reporter gene is a luciferase gene, by detecting the fluorescence of a fluorescent compound caused by the catalytic action of the gene expression product, in the case where the reporter gene is a β-glucuronidase gene (GUS), by detecting the luminescence of Glucuron (ICN) or the coloring of 5-bromo-4-chloro-3-indolyl-β-glucuronide (X-Gluc) by the catalytic action of the gene expression product, and in the case where the reporter gene is a GFP gene, by detecting the fluorescence of a GFP protein, the expression level of the reporter gene can be measured.

Further, in the case where a melanin-concentrating hormone receptor gene is used as the reporter, the expression level of the gene can be measured by the method described in the second embodiment.

In the third embodiment, subsequently, the test compound which decreases the expression level of the reporter gene in comparison with the case where the test compound is not brought into contact is selected. In the selected compounds, a compound which decreases the expression level of the reporter gene is included, and a compound which decreases the expression of the melanin-concentrating hormone receptor is included.

In a fourth embodiment of the screening method of the invention, first, a test compound is brought into contact with a cell which expresses a melanin-concentrating hormone receptor on a cell surface in the presence of a ligand for the melanin-concentrating hormone receptor.

The "ligand" to be used in this description refers to a molecule such as a random peptide or a variable segment sequence that is recognized by a specific receptor. The molecule (or a macromolecular complex) as recognized by those skilled in the art can be both receptor and ligand. In general, a binding partner having a smaller molecular weight is referred to as a ligand, and a binding partner having a larger molecular weight is referred to as a receptor. Specific examples of the ligand include melanin-concentrating hormones.

In the forth embodiment, subsequently, the activity of the melanin-concentrating hormone receptor is measured. Then, the test compound which decreases the activity thereof in comparison with the case where the test compound is not brought into contact is selected. In the selected compounds, a compound which decreases the activity of the melanin-concentrating hormone receptor is included. In this connection, because the melanin-concentrating hormone receptor is a G-protein conjugated receptor, in the activity of the melanin-concentrating hormone receptor, a GTP binding ability of G-protein to be conjugated is included, and further, the activity of intracellular signal transduction system is also included. Specific examples of the activity of intracellular signal transduction system include calcium influx, inhibition of cAMP, and activation of MAP kinase. These can be measured by a known method in any case.

In the screening method of the invention, the melanin-concentrating hormone receptor is preferably melanin-concentrating hormone receptor 1.

### Examples

### (Example 1)

Male mice (C57BL/6J, Nippon CLEA) were fed with MHF diet (a moderately high fat diet, Oriental Bioservice Kanto), which is a high-calorie diet, to produce a model mouse with obesity (diet-induced obesity mouse: DIO mouse), and an effect of Compound A which is an MCH1R antagonist on the liver weight and plasma ALT level of the DIO mice was examined. The inhibition constants (Ki) of Compound A for MCH1R and MCH2R are 9.9 nM and >9400 nM, respectively. The structure of Compound A is H₂N-Cys-Ava-Tyr-Val-Arg- Ava-Met-Cys-Arg-C(=O)CH₃ (Ava represents 5-aminovaleric acid, two Cys residues are bound to each other through a -SS- bond).

A sterilized brain infusion cannula (Durect Corporation) was implanted stereotaxically in the right lateral ventricle of mice at 26 to 27 weeks of age under pentobarbital anesthesia (80 mg/kg, i.p., Dainabot). The cannula was fixed vertically to the skull with dental cement at coordinates of 0.4 mm posterior, 0.8 mm lateral and 0.2 mm deep to the bregma. The cannula was connected to an osmotic pump (Model No. 2004, Durect Corporation) filled with 30% propylene glycol (30% PG) via a polyvinyl chloride tube. The pump was embedded under the skin of the back of the mouse. In order to prevent infection of the mice, an antibiotic (Cefamezin α, 50 mg/kg, Fujisawa Pharmaceutical Company, Ltd.) was subcutaneously administered.

After a sufficient period of time for recovery (1 to 2 weeks) from the insertion of cannula had passed, the mice were divided into an MHF diet group and a normal diet (CE-2, Nippon CLEA) group. In the MHF diet group, the mice were divided such that the body weights became equal between groups. The numbers of mice allocated to the respective groups are as follows. Incidentally, the mice were raised with the normal diet until they were divided into groups.
MHF diet and vehicle administration group: 14
MHF diet and Compound A administration group: 14
Normal diet group (vehicle administration): 5

The administration of an agent was carried out according to the following procedure. A new osmotic pump was filled with a vehicle (30% PG, distilled water solution) or a Compound A (7.5 µg/day, 1.25 mg/mL, 0.25 µL/hour) solution subjected to filter sterilization (0.22 µm). Replacement of the osmotic pump was carried out under isoflurane anesthesia, and then, administration of the agent to the ventricle was initiated.

The mice were subjected to thoracotomy under isoflurane anesthesia, and the blood was collected from the heart using a syringe containing heparin. The collected blood was centrifuged for 10 minutes (4°C, 6000 rpm), and the plasma was separated. The obtained plasma was stored at -80°C until a biochemical parameter was measured. Then, the liver was excised and the wet weight was measured.

The biochemical parameter measured for the plasma sample from the heart is ALT (measured with HITACHI Clinical analyzer 7070 (Hitachi Co., Ltd.)).

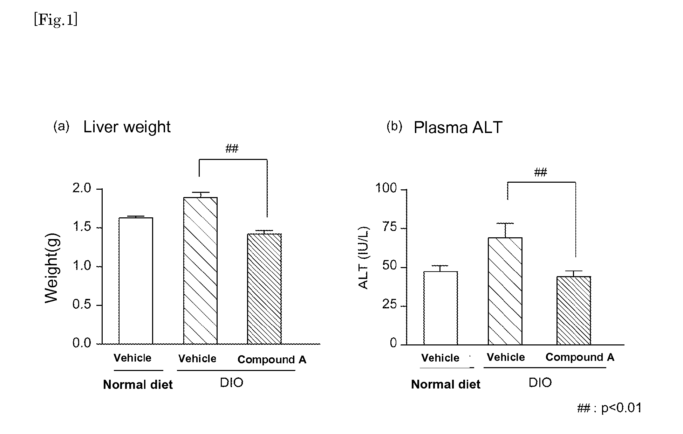

The analysis results are shown in Fig. 1. Fig. 1(a) shows the liver weight, and Fig. 1(b) shows the plasma ALT. It was found that when Compound A was administered to DIO mice, both of the liver weight and the plasma ALT decrease to a normal level (the same level as that of the mice fed with the normal diet).

### (Example 2)

Male mice (C57BL/6J, Nippon CLEA) were fed with MCD diet (a methionine choline deficient diet) to produce mice in which NASH was induced, and an effect of Compound A on the NASH mice was examined.

In order to intraventricularly administer an agent, a cannula was inserted into mice at 16 to 17 weeks of age in the same manner as in Example 1. Incidentally, mice which were continued to be raised with a normal diet (CE-2) were subjected to sham surgery (only incision and suture of the dorsal skin).

After a sufficient period of time for recovery (1 to 2 weeks) from the insertion of cannula had passed, the mice were divided such that the body weights became equal between groups. The numbers of mice allocated to the respective groups are as follows. Incidentally, the mice were raised with the normal diet (CE-2) and at 4 days after initiation of administration of an agent, the diet was changed to a predetermined diet.
Pellet-type MCD diet and vehicle administration group: 12
Pellet-type MCD diet and Compound A administration group: 12
Pellet-type control diet and vehicle administration group: 11
Pellet-type control diet and Compound A administration group: 11
Normal diet and sham surgery group: 7

The intraventricular administration of Compound A was carried out in the same manner as in Example 1. Also, the vehicle was administered in the same manner as in Example 1. As the pellet-type MCD diet and control diet, ICN 960439 and ICN 960441 available from ICN Biomedicals were used, respectively.

On day 11 after initiation of loading of the MCD diet, the blood and organs of the mice were collected in the same manner as in Example 1. The biochemical parameters measured for the plasma sample from the heart were AST and ALT, which were measured with HITACHI Clinical analyzer 7070 (Hitachi Co., Ltd.).

Further, a portion of the liver was excised during dissection, and measurement of hepatic biochemical parameters, measurement of hepatic mRNA, and histopathological observation were carried out. The measurement of hepatic biochemical parameters and measurement of mRNA were carried out according to the following procedure. One lobe of the liver was excised and after the weight thereof was measured, it was cryopreserved. The cryopreserved one lobe of the liver was homogenized, and a lipid fraction was extracted from a portion of the resulting homogenate with Folch reagent. Then, the extracted lipid fraction was dried and hardened with nitrogen gas, and the triglyceride was measured using Determiner L TG II (Kyowa Medex). Further, by using a portion of the homogenate, TBARS (thiobarbituric acid reactive substance, one of the markers for oxidative stress), which is a parameter of lipid peroxide, was measured with reference to the method described in Method in Enzymology, vol. 186, p. 407 (1990). From the portion of the liver (50 mg) collected for measurement of mRNA, RNA was extracted using ISOGEN (NIPPON GENE), and cDNA was synthesized using Taqman RT reagents (Applied Biosystems). By using Taqman real time PCR (HT7900, Applied Biosystems), TNFα and IL-1β, which are inflammatory cytokines, and the expression level of Cyp4A14, which is involved in the formation of lipid peroxide, were measured (the expression level was represented by a ratio to 18s rRNA). The histopathological observation was carried out according to the following procedure. The middle lobe of the liver was excised and fixed in a 10% neutral buffered formalin solution. A paraffin section was prepared according to a standard method, and HE staining and fat staining were carried out. Histopathological evaluation of tissue lesions including inflammatory cell infiltration and hepatocellular vacuolation (fatty change) was carried out by using the degree of the change and range of the distribution as indices.

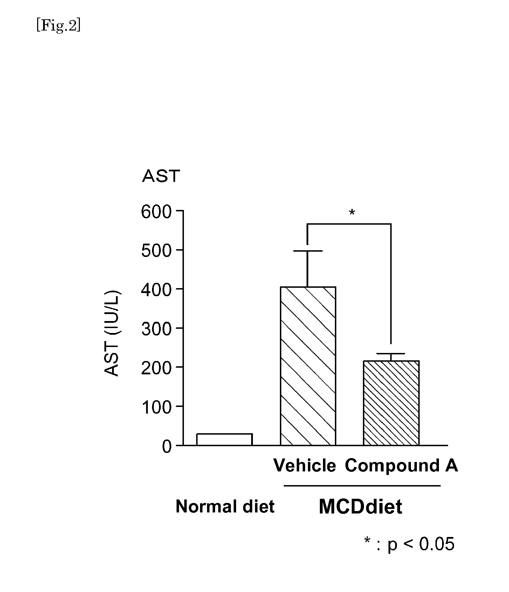

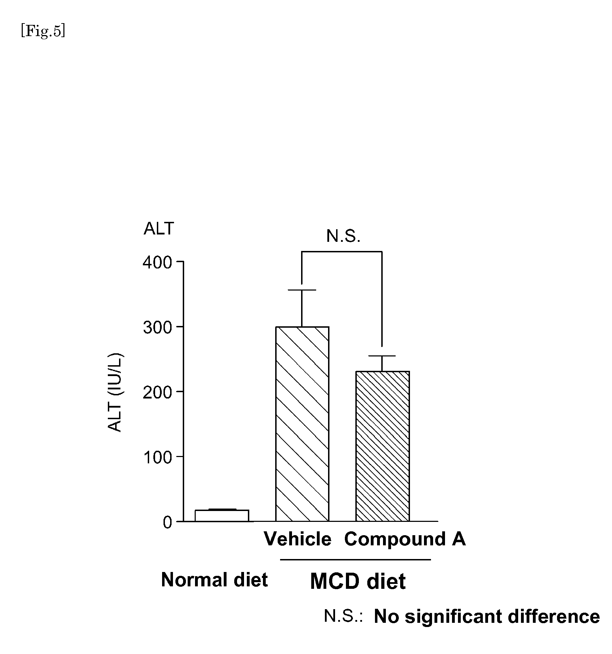

The results are shown in Figs. 2 to 7. Fig. 2 shows the plasma AST level, and Fig. 5 shows the plasma ALT level. By the loading of MCD, the plasma AST level was significantly increased, however, by the administration of Compound A, the increase thereof was suppressed. Further, a similar tendency was observed with regard also to the ALT.

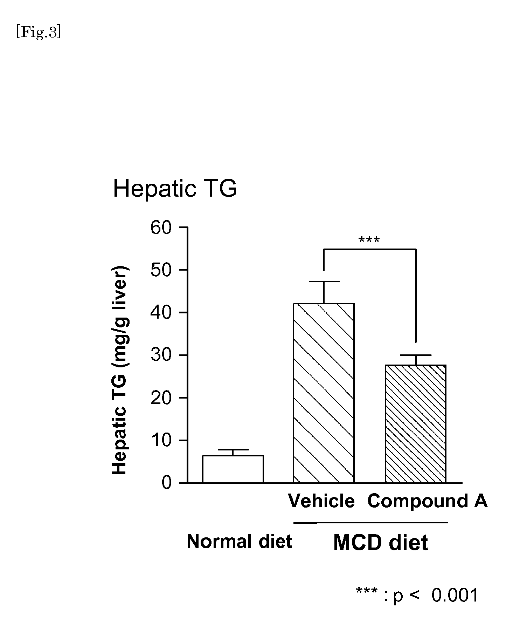

Fig. 3 shows the hepatic triglyceride level. The hepatic triglyceride increased by the MCD diet was suppressed by the administration of Compound A. Accordingly, it was found that Compound A alleviates fatty liver which is a key factor of occurrence of NASH.

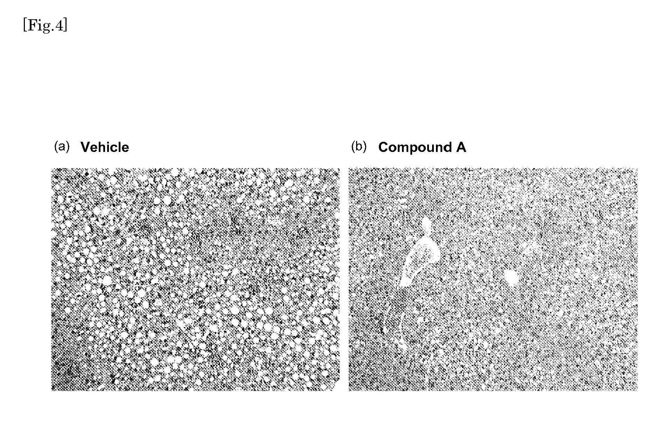

Fig. 4 shows typical images of liver pathology; Fig. 4(a) shows a vehicle administration group, and Fig. 4(b) shows a Compound A administration group. In the vehicle administration group, lipid droplets and inflammatory cell infiltration were observed. However, in the Compound A administration group, it was observed that both were reduced. Further, the observation results of multifocal cell infiltration and single cell necrosis are summarized in Table 1. It was found that an effect on improving both fatty liver and inflammation can be obtained by the administration of Compound A.

**[Table 1]**

| | MCD diet | |
|---|---|---|
| | Vehicle (n = 11) | Compound A (n = 10) |
| Multifocal cell infiltration | | |
| Very slight | 2 | 6 |
| Slight | 6 | 4 |
| Moderate | 2 | 0 |
| Single cell necrosis | | |
| Very slight | 8 | 10 |
| Slight | 2 | 0 |

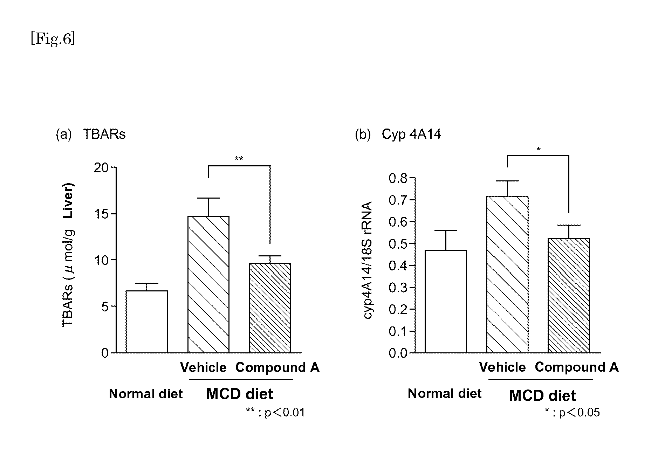

Fig. 6(a) shows the TBARS level in the liver, and Fig. 6(b) shows the expression level of Cyp4A14 in the liver. The TBARS, which is a parameter of lipid peroxide, was increased by the MCD diet, however, it was suppressed by the administration of Compound A. Further, the expression of Cyp4A14 which is involved in the formation of lipid peroxide was induced by the MCD diet, however, the expression thereof was decreased by the administration of Compound A.

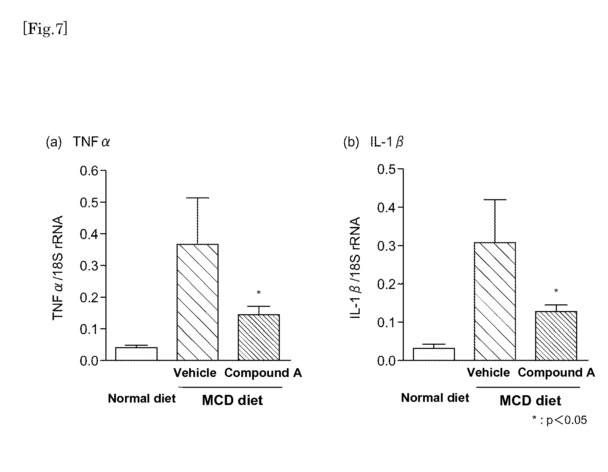

Fig. 7(a) shows the expression level of TNFα in the liver, and Fig. 7(b) shows the expression level of IL-1β in the liver. The expression of TNFα and IL-1β, which are inflammatory cytokines, was induced by the MCD diet, however, the expression thereof was decreased by the administration of Compound A. Thus, it was strongly suggested that hepatitis is relieved.

### (Example 3)

The inhibitory action of Compound A against various receptors listed in Table 2 was examined to evaluate the specificity of Compound A. The activities of the receptors were measured by using an appropriate assay system according to the property of the respective receptors. Further, the evaluation results of Compound A were calculated as an inhibition ratio at a final concentration of 10 µM. Here, the inhibition ratio was calculated based on a value of a control compound in each assay. As is apparent from the results shown in Table 2, it was confirmed that Compound A is specific to an MCH receptor. Incidentally, examination was carried out with regard to 173 kinds of physiologically functional proteins including the receptors shown in Table 2, however, the affinity thereof could not be found other than the MCH receptors, and only major receptors are shown in Table 2.

**[Table 2]**

| Receptor | Inhibition ratio |
|---|---|
| Glutamate AMPA | 8 |
| Histamine H3 | -6 |
| Muscarine M1 | 0 |
| Neuropeptide Y1 | 17 |

### (Example 4)

Male mice (C57BL/6J, Nippon CLEA) were fed with HFD (a high fat diet, D12492, Research Diets Inc.) for about 1 year. The obtained mice were used as an NASH pathological model, and an effect of Compound A on the NASH mice was examined.

The mice were fed with HFD or a normal diet (CE-2). During the test period, the feed and water were given ad libitum, and the mice were not subjected to fasting.

In order to intraventricularly administer an agent, a cannula was inserted into mice at 62 weeks of age in the same manner as in Example 1. Incidentally, an antibiotic used was 100 mg/kg of Cefamezin α.

The measurement of the body weight, amount of water intake, and amount of food intake was carried out for 1 week before initiation of administration of an agent, and the mice were divided such that these data became equal between groups. The numbers of mice allocated to the respective groups are as follows.
HFD and vehicle administration group: 6
HFD and Compound A administration group: 6
Normal diet and vehicle administration group: 6

The intraventricular administration of Compound A was carried out in the same manner as in Example 1. Also, the vehicle was administered in the same manner as in Example 1.

At 4 weeks after the initiation of administration of an agent, the blood and organs of the mice were collected in the same manner as in Example 1. Incidentally, before the dissection of mice, the body fat percentage was measured using NMR (Minispec mq 7.5, Bruker Optics). The hepatic triglyceride, ALT and AST were measured in the same manner as in Example 2. Further, histopathological observation of the liver was carried out in the same manner as in Example 2.

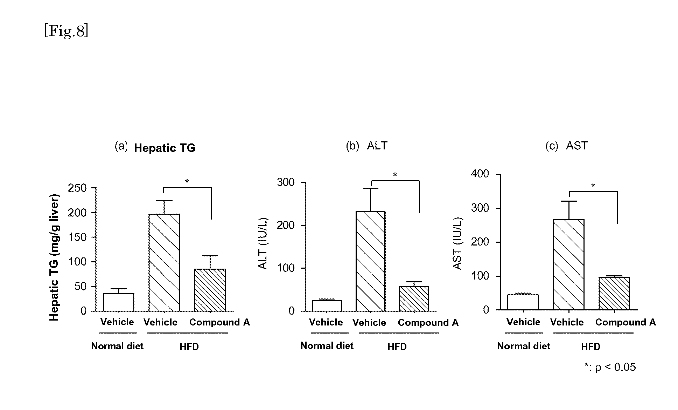

Fig. 8(a) shows the hepatic triglyceride level, Fig. 8(b) shows the plasma ALT level, and Fig. 8(c) shows the plasma AST level. By the loading of HFD, the hepatic triglyceride level, plasma ALT level, and plasma AST level were significantly increased, however, by the administration of Compound A, the increase thereof was suppressed. It was found that Compound A alleviates fatty liver which is a key factor of occurrence of NASH.

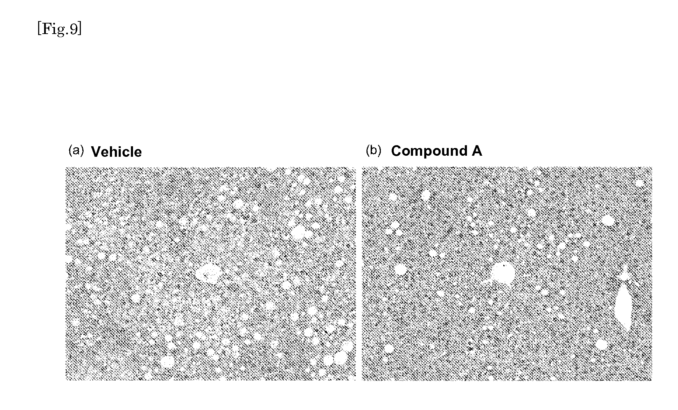

Fig. 9 shows typical images of liver pathology; Fig. 9(a) shows a vehicle administration group, and Fig. 9(b) shows a Compound A administration group. In the vehicle administration group, lipid droplets and inflammatory cell infiltration were observed. However, in the Compound A administration group, it was observed that both were reduced. Further, the observation results of multifocal cell infiltration, single cell necrosis and hepatocellular vacuolation are summarized in Table 3. It was found that an effect on improving both fatty liver and inflammation can be obtained by the administration of Compound A.

**[Table 3]**

| | HF diet | |
|---|---|---|
| | Vehicle (n = 6) | Compound A (n = 6) |
| Multifocal cell infiltration | | |
| Very slight | 3 | 3 |
| Slight | 3 | 0 |
| Single cell necrosis | | |
| Very slight | 4 | 0 |
| Hepatocellular vacuolation | | |
| Very slight | 0 | 1 |
| Slight | 0 | 2 |
| Moderate | 3 | 3 |
| Significant | 3 | 0 |

### INDUSTRIAL APPLICABILITY

Because a therapeutic agent for NAFLD based on a novel mechanism of action, the choice of treatment of NAFLD is expanded.

## Claims

1. The use of a melanin-concentrating hormone receptor antagonist for the manufacture of a medicament for the treatment or prevention of non-alcoholic fatty liver disease.

2. The use according to claim 1, wherein the non-alcoholic fatty liver disease is non-alcoholic fatty liver.

3. The use according to claim 1, wherein the non-alcoholic fatty liver disease is non-alcoholic steatohepatitis.

4. The use according to any one of claims 1 to 3, wherein the melanin-concentrating hormone receptor antagonist is a melanin-concentrating hormone receptor 1 antagonist.

5. A method for screening a drug candidate compound for the treatment or prevention of a non-alcoholic fatty liver disease comprising the steps of:
(a) bringing a test compound into contact with a melanin-concentrating hormone receptor;
(b) detecting binding of the test compound to the melanin-concentrating hormone receptor; and
(c) selecting the test compound binding to the melanin-concentrating hormone receptor.

6. A method for screening a drug candidate compound for the treatment or prevention of a non-alcoholic fatty liver disease comprising the steps of:
(a) bringing a test compound into contact with a cell which expresses a melanin-concentrating hormone receptor;
(b) measuring the expression level of the melanin-concentrating hormone receptor; and
(c) selecting the test compound which decreases the expression level of the melanin-concentrating hormone receptor in comparison with the case where the test compound is not brought into contact.

7. A method for screening a drug candidate compound for the treatment or prevention of a non-alcoholic fatty liver disease comprising the steps of:
(a) providing a cell or a cell extract having a DNA in which a reporter gene is functionally linked downstream of a promoter region of a DNA encoding a melanin-concentrating hormone receptor;
(b) bringing a test compound into contact with the cell or cell extract;
(c) measuring the expression level of the reporter gene in the cell or cell extract; and
(d) selecting the test compound which decreases the expression level of the reporter gene in comparison with the case where the test compound is not brought into contact.

8. A method for screening a drug candidate compound for the treatment or prevention of a non-alcoholic fatty liver disease comprising the steps of:
(a) bringing a test compound into contact with a cell which expresses a melanin-concentrating hormone receptor on a cell surface in the presence of a ligand for the melanin-concentrating hormone receptor;
(b) measuring the activity of the melanin-concentrating hormone receptor in the cell; and
(c) selecting the test compound which decreases the activity of the melanin-concentrating hormone receptor in comparison with the case where the test compound is not brought into contact.

9. The screening method according to any one of claims 5 to 8, wherein the non-alcoholic fatty liver disease is non-alcoholic fatty liver.

10. The screening method according to any one of claims 5 to 8, wherein the non-alcoholic fatty liver disease is non-alcoholic steatohepatitis.

11. The screening method according to any one of claims 5 to 10, wherein the melanin-concentrating hormone receptor is melanin-concentrating hormone receptor 1.
